# EUROPÄISCHE PATENTSCHRIFT

(11) **EP 1 645 582 B1**
(45) Veröffentlichungstag und Bekanntmachung des Hinweises auf die Patenterteilung: **03.08.2011**
(21) Anmeldenummer: 05020859.4
(22) Anmeldetag: 24.09.2005
(51) Int. Cl.: C08G 18/78, C09D 175/16

(54) **Verfahren zur Herstellung von niedrigviskosen Allophanaten mit aktinisch härtbaren Gruppen**
Procédé de preparation d'allophanates possédant une faible viscosité et des groupes durcissables sous rayonnement actinique
Process for preparing low viscosity allophanates having actinically curable groups

(30) Priorität: 07.10.2004 DE 102004048873
(43) Veröffentlichungstag der Anmeldung: 12.04.2006
(73) Patentinhaber: Bayer MaterialScience AG, 51368 Leverkusen (DE)
(72) Erfinder: Ludewig, Michael, Dr., 51065 Köln (DE); Weikard, Jan, Dr., 51519 Oldenthal (DE)

(56) Entgegenhaltungen:
- EP-A- 0 867 457
- EP-A- 0 899 283
- WO-A-2005/092942
- DE-A1- 10 246 512
- DE-A1- 19 860 041

## Beschreibung

Die vorliegende Erfindung betrifft ein vereinfachtes Verfahren zur Herstellung von niedrigviskosen Umsetzungsprodukten von Polyisocyanaten, die aktivierte unter Einwirkung aktinischer Strahlung mit ethylenisch ungesättigten Verbindungen unter Polymerisation reagierende Gruppen enthalten.

Die Härtung von aktivierte Doppelbindungen tragenden Beschichtungssystemen durch aktinische Strahlung, wie z. B. UV-Licht, IR-Strahlung oder auch Elektronenstrahlung ist bekannt und technisch etabliert. Es ist eine der schnellsten Härtungsmethoden in der Beschichtungstechnologie. Auf diesem Prinzip basierende Beschichtungsmittel werden daher als strahlen- oder aktinisch härtende bzw. härtbare Systeme bezeichnet.

Bedingt durch die ökologischen und ökonomischen Anforderungen an moderne Lacksysteme möglichst wenig oder gar kein organisches Lösungsmittel zur Viskositätseinstellung zu verwenden, besteht der Wunsch, bereits niedrigviskose Lackrohstoffe zu verwenden. Bekannt hierfür sind seit langem Polyisocyanate mit Allophanatstruktur wie sie u. a. in EP-A 0 682 012 beschrieben werden.

In der Technik werden diese durch Umsetzung eines ein- oder mehrwertigen Alkohols mit großen Mengen an überschüssigem aliphatischen und/oder cycloaliphatischen Diisocyanat hergestellt (vgl. GB-A 994 890, EP-A 0 000 194 oder EP-A 0 712 840). Anschließend erfolgt die Entfernung nicht reagierten Diisocyanats mittels Abdestillation im Vakuum. Nach DE-A 198 60 041 kann diese Verfahrensweise auch mit OH-funktionellen Verbindungen mit aktivierten Doppelbindungen, wie z.B. Hydroxyalkylacrylaten, durchgeführt werden, wobei jedoch zur Herstellung besonders monomerenarmer Produkte Schwierigkeiten auftreten. Da der Destillationsschritt bei Temperaturen bis zu 135°C verlaufen muss, um den Rest-Isocyanatgehalt ausreichend senken zu können (< 0,5 Gew.-% Restmonomer), können während der Aufreinigung bereits Doppelbindungen thermisch initiiert unter Polymerisation reagieren, so dass keine einwandfreien Produkte mehr erhalten werden.

Die Herstellung monomerenarmer, allophanathaltiger, strahlenhärtender Bindemittel auf Polyurethanbasis wird in EP-A 0 867 457 und US-A 5 739 251 beschrieben. Diese Bindemittel tragen allerdings keine aktivierten Doppelbindungen, sondern unreaktive Allylethergruppen (Struktur R-O-CH₂-CH=CH₂). Daher benötigt man den Zusatz von Reaktiwerdünnern (niedermolekulare Ester der Acrylsäure), die die notwendige UV-Reaktivität einbringen.

Ebenso hat es nicht an Versuchen gemangelt, Allophanate indirekt, aus anderen Isocyanatderivaten, als Urethanen und Isocyanaten herzustellen. So beschreibt die EP-A 0 825 211 ein Verfahren zum Aufbau von Allophanatstrukturen aus Oxadiazintrionen, wobei hier allerdings keine strahlenhärtenden Derivate mit aktivierten Doppelbindungen erwähnt werden. Die Übertragung auf die besonderen Gegebenheiten von strahlenhärtenden Systemen wird in DE-A-102 46 512 beschrieben.

Ein weiterer Weg ist die Öffnung von Uretdionen (vgl. Proceedings of the International Waterborne, High-Solids, and Powder Coatings Symposium 2001, 28^{th}, 405-419 sowie US-A 2003 0153713) zu Allophanat-Strukturen, der ebenfalls schon erfolgreich auf strahlenhärtende Systeme übertragen werden konnte (DE-A-102004012903).

Beide Wege benötigen veredelte Rohstoffe als Ausgangsmaterial und führen nur zu einem an Nebenprodukten reichen Allophanatprodukt.

US-A 5 777 024 beschreibt die Herstellung von niedrigviskosen strahlenhärtenden Allophanaten durch eine Reaktion von hydroxyfunktionellen Monomeren, die aktivierte Doppelbindungen tragen, mit Isocyanatgruppen von bereits allophanatmodifizierten Isocyanurat-Polyisocyanaten. Die über die Allophanatgruppen gebundenen Reste sind dabei gesättigt, wodurch auf eine mögliche höhere Funktionalität verzichtet wird.

Aus EP-B 694 531 wird ein mehrstufiges Verfahren zur Herstellung hydrophilierter Allophanate mit strahlenhärtenden Gruppen beschrieben. Dabei wird jedoch zunächst ein NCO- und acrylatfunktionelles Urethan hergestellt, welches dann hydrophiliert wird und anschließend nach Zugabe eines weiteren NCO- und acrylatfunktionellen Urethans allophanatisiert wird. Als Verfahrenstemperatur zur Allophanatisierung werden Temperaturen von 100 bis 110°C angegeben.

Es war nun die Aufgabe der vorliegenden Erfindung auf Basis von leicht verfügbaren Rohstoffen in einem Prozess bereits bei moderater Temperatur von unter 100 °C ohne Destillationschritt ein NCOgruppenfreies, hochfunktionelles Allophanatgemisch mit durch aktinische Strahlung vernetzbaren Gruppen (strahlenhärtenden Gruppen) als strahlenhärtendes Bindemittel zur Verfügung zu stellen, wobei dieses einen Restgehalt an Diisocyanatmonomeren von weniger als 0,5 Gew.% aufweisen soll. Die Viskosität dieses Produktes soll dabei so niedrig sein, d.h. unterhalb von 200 000 mPas @ 23°C, dass eine Verarbeitung bei Raumtemperatur auch ohne Lösemittelzusatz möglich ist.

Es wurde nun gefunden, dass sich solche strahlenhärtenden Allophanate gerade dann herstellen lassen, wenn man bestimmte NCO/OH-Verhältnisse bei der Herstellung einhält.

Gegenstand der Erfindung ist ein Verfahren zur Herstellung von strahlenhärtenden Allophanaten mit Restmonomergehalten von weniger als 0,5 Gew.-% und einem NCO-Gehalt von weniger als 1 Gew.%, bei dem aus
A) isocyanatgruppenhaltigen Verbindungen,
B) hydroxyfunktionellen Verbindungen, die unter Einwirkung aktinischer Strahlung mit ethylenisch ungesättigten Verbindungen unter Polymerisation reagierende Gruppen (strahlenhärtende Gruppen) aufweisen und
C) gegebenenfalls weitere Verbindungen mit NCO-reaktiven Gruppen
D) gegebenenfalls in Anwesenheit eines Katalysators
   NCO-gruppenhaltige Urethane mit strahlenhärtenden Gruppen gebildet werden, die anschließend ohne weitere Zugabe an isocyanatgruppenhaltigen Verbindungen in Anwesenheit
E) eines Allophanatisierungskatalysators umgesetzt werden,
   wobei das Verhältnis von NCO-Gruppen der Verbindungen aus A) zu den OH-Gruppen von den Verbindungen aus B) und ggf. C) 1,45 : 1,0 bis 1,1 : 1,0 beträgt.

Darüber hinaus sind die nach dem erfindungsgemäßen Verfahren erhältlichen Bindemittel ein Gegenstand der Erfindung.

Bevorzugt beträgt das Verhältnis von NCO-Gruppen der Verbindungen aus A) zu den OH-Gruppen von den Verbindungen aus B) und ggf. C) 1,43 : 1,0 bis 1,2 : 1,0, besonders bevorzugt 1,35 : 1,0 bis 1,3 : 1,0.

Als isocyanathaltige Verbindungen A) kommen aromatische, aliphatische und cycloaliphatische Polyisocyanate in Betracht. Geeignete Polyisocyanate sind Verbindungen der Formel Q(NCO)ₙ mit einem zahlenmittleren Molekulargewicht unter 800 g/mol, worin n eine Zahl von 2 bis 4 und Q einen aromatischen C₆-C₁₅-Kohlenwasserstoffrest, einen aliphatischen C₄-C₁₂-Kohlenwasserstoffrest oder einen cycloaliphatischen C₆-C₁₅-Kohlenwasserstoffrest bedeuten. Beispielsweise geeignet sind Diisocyanate aus der Reihe 2,4-/2,6-Toluoldiisocyanat (TDI), Methylendiphenyldiisocyanat (MDI), Triisocyanatononan (TIN), Naphtyldiisocyanat (NDI), 4,4'-Diisocyanatodicyclohexylmethan, 3-Isocyanatomethyl-3,3,5-trimethylcyclohexylisocyanat (Isophorondiisocyanat = IPDI), Tetramethylendiisocyanat, Hexamethylendiisocyanat (HDI), 2-Methyl-pentamethylendiisocyanat, 2,2,4-Trimethylhexamethylendiisocyanat (THDI), Dodecamethylendiisocyanat, 1,4-Diisocyanatocyclohexan, 4,4'-Diisocyanato-3,3'-dimethyl-dicyclohexylmethan, 4,4'-Diisocyanatodicyclohexylpropan-(2,2), 3-Isocyanatomethyl-1-methyl-1-isocyanatocyclohexan (MCI), 1,3-Diisooctylcyanato-4-methyl-cyclohexan, 1,3-Diisocyanato-2-methyl-cyclohexan und α,α,α',α'-Tetramethyl-m- oder -p-xylylen-diisocyanat (TMXDI) sowie aus diesen Verbindungen bestehende Gemische.

Ebenfalls geeignet als isocyanathaltige Verbindungen A) sind Umsetzungsprodukte der vorgenannten Isocyanate mit sich selbst oder untereinander zu Uretdionen oder Isocyanuraten. Beispielhaft genannt seien Desmodur® N3300, Desmodur® N3400 oder Desmodur® N3600 (alle Bayer MaterialScience, Leverkusen, DE).

Weiterhin geeignet als isocyanathaltige Verbindungen A) sind Umsetzungsprodukte der vorgenannten Isocyanate mit anderen isocyanatreaktiven Verbindungen zu Prepolymeren. Solche isocyanatreaktiven Verbindungen sind vor allem Polyole, wie z.B. Polyetherpolyole, Polyesterpolyole, Polycarbonatpolyole und mehrwertige Alkohole. Als Polyole können höhermolekulare und in untergeordneter Menge auch niedermolekulare Hydroxylverbindungen eingesetzt werden.

Die Verbindungen der Komponente A) können dementsprechend direkt in das erfindungsgemäße Verfahren eingesetzt werden oder, ausgehend von einer beliebigen Vorstufe durch Vorreaktion hergestellt werden, bevor das erfindungsgemäße Verfahren durchgeführt wird.

Bevorzugt als Komponente A) ist die Verwendung von monomeren Diisocyanaten. Ganz besonders bevorzugt ist die Verwendung von Hexamethylendiisocyanat (HDI), Isophorondiisocyanat (IPDI) und/oder 4,4'-Diisocyanatodicyclohexylmethan.

Unter aktinischer Strahlung wird elektromagnetische, ionisierende Strahlung verstanden, insbesondere Elektronenstrahlen, UV-Strahlen sowie sichtbares Licht (Roche Lexikon Medizin, 4.Auflage; Urban & Fischer Verlag, München 1999).

Unter Einwirkung aktinischer Strahlung mit ethylenisch ungesättigten Verbindungen unter Polymerisation reagierende Gruppen (strahlenhärtende Gruppen) werden im Rahmen der vorliegenden Erfindung Vinylether-, Maleinyl-, Fumaryl-, Maleinimid-, Dicyclopentadienyl-, Acrylamid-, Acryl- und Methacryl-Gruppen verstanden, wobei dies bevorzugt Vinylether-, Acrylat- und/oder Methacrylat-Gruppen, besonders bevorzugt Acrylatgruppen sind.

Beispielsweise geeignete hydroxylgruppenhaltige Verbindungen der Komponente B) sind 2-Hydroxyethyl(meth)acrylat, Polyethylenoxid-mono(meth)acrylat (z.B. PEA6/PEM6; Laporte Performance Chemicals Ltd., UK), Polypropylenoxidmono(meth)acrylat (z.B. PPA6, PPM5S; Laporte Performance Chemicals Ltd., UK), Polyalkylenoxidmono(meth)acrylat (z.B. PEM63P, Laporte Performance Chemicals Ltd. , UK), Poly(ε-caprolacton)mono(meth)acrylate wie z.B. Tone M100® (Dow, Schwalbach, DE), 2-Hydroxypropyl(meth)acrylat, 4-Hydroxybutyl(meth)acrylat, Hydroxybutylvinylether, 3-Hydroxy-2,2-dimethylpropyl(meth)acrylat, die hydroxyfunktionellen Mono-, Di- oder soweit möglich höheren Acrylate wie z.B. Glycerindi(meth)acrylat, Trimethylolpropandi(meth)acrylat, Pentaerythrithi(meth)acrylat oder Dipentaerythritpenta(meth)acrylat, die durch Umsetzung mehrwertiger gegebenenfalls alkoxylierter Alkohole wie Trimethylolpropan, Glycerin, Pentaerythrit, Dipentaerythrit zugänglich sind.

Ebenfalls geeignet als Bestandteil von B) sind auch Alkohole, die sich aus der Umsetzung von doppelbindungshaltigen Säuren mit gegebenenfalls doppelbindungshaltigen Epoxidverbindungen erhalten werden, so z.B. die Umsetzungsprodukte von (Meth)acrylsäure mit Glycidyl(meth)acrylat oder Bisphenol-A-diglycidylether.

Darüber hinaus können ebenfalls ungesättigte Alkohole eingesetzt werden, die aus der Umsetzung von gegebenenfalls ungesättigten Säureanhydriden mit gegebenenfalls acrylatgruppenhaltigen Hydroxy- und Epoxidverbindungen erhalten werden. Beispielsweise sind dies die Umsetzungsprodukte von Maleinsäureanhydrid mit 2-Hydroxyethyl(meth)acrylat und Glycidyl(meth)-acrylat.

Besonders bevorzugt entsprechen die Verbindungen der Komponente B) der vorgenannten Art und weisen eine OH-Funktionalität von 0,9 bis 1,1 auf.

Bevorzugt ist die Verwendung von Hydroxyethyl(meth)acrylat, Hydroxypropyl(meth)acrylat und Hydroxybutyl(meth)acrylat. Ganz besonders bevorzugt sind Hydroxyethylacrylat und Hydroxypropylacrylat.

Neben den OH-funktionellen ungesättigten Verbindungen der Komponente B) können im erfindungsgemäßen Verfahren auch weitere Verbindungen C) eingesetzt werden, die verschieden von denen aus B) sind und NCO-reaktive Gruppen wie beispielsweise OH, SH oder NH aufweisen.

Dies können beispielsweise NH- oder SH-funktionelle Verbindungen mit unter Einwirkung aktinischer Strahlung mit ethylenisch ungesättigten Verbindungen unter Polymerisation reagierende Gruppen sein.

Auch unter Einwirkung von aktinischen Strahlen nichtreaktive Verbindungen, wie z.B. Polyetherpolyole, Polyesterpolyole, Polycarbonatpolyole und mehrwertige Alkohole können zur Beeinflussung der Produkteigenschaften als Komponente C) mitverwendet werden. Als Polyole können höhermolekulare und in untergeordneter Menge auch niedermolekulare Hydroxylverbindungen eingesetzt werden.

Höhermolekulare Hydroxylverbindungen umfassen die in der Polyurethan-Chemie üblichen Hydroxypolyester, Hydroxypolyether, Hydroxypolythioether, Hydroxypolyacetale, Hydroxypolycarbonate, Dimerfettalkohole und/oder Esteramide, jeweils mit mittleren Molekulargewichten von 400 bis 8 000 g/mol, bevorzugt solche mit mittleren Molekulargewichten von 500 bis 6 500 g/mol. Bevorzugte höhermolekulare Hydroxylverbindungen sind Hydroxypolyether, Hydroxypolyester und Hydroxypolycarbonate.

Als niedermolekulare Polyhydroxylverbindungen können in der Polyurethan-Chemie übliche Polyole, mit Molekulargewichten von 62 bis 399, wie Ethylenglykol, Triethylenglykol, Tetraethylenglykol, Propandiol-1,2 und -1,3, Butandiol-1,4 und -1,3, Hexandiol-1,6, Octandiol-1,8, Neopentylglykol, 1,4-Bis(hydroxymethyl)cyclohexan, Bis(hydroxymethyl)tricyclo[5.2.1.0^{2.6}]decan oder 1,4-Bis(2-hydroxyethoxy)benzol, 2-Methyl-1,3-propandiol, 2,2,4-Trimethylpentandiol, 2-Ethyl-1,3-hexandiol, Dipropylenglykol, Polypropylenglykole, Dibutylenglykol, Polybutylenglykole, Bisphenol A, Tetrabrombisphenol A, Glycerin, Trimethylolpropan, Hexantriol-1,2,6-Butantriol-1,2,4, Pentaerythrit, Chinit, Mannit, Sorbit, Methylglykosid und 4,3,6-Dianhydrohexite verwendet werden.

Als Polyetherpolyole geeignet sind die in Polyurethan-Chemie üblichen Polyether, wie z.B. die unter Verwendung von zwei- bis sechswertigen Startermolekülen wie Wasser oder den oben genannten Polyolen oder 1- bis 4-NH-Bindungen aufweisenden Aminen hergestellten Additions- bzw. Mischadditionsverbindungen des Tetrahydrofurans, Styroloxids, Ethylenoxids, Propylenoxids, der Butylenoxide oder des Epichlorhydrins, insbesondere des Ethylenoxids und/oder des Propylenoxids. Bevorzugt sind im Durchschnitt 2 bis 4 Hydroxylgruppen aufweisende Propylenoxidpolyether, die bis zu 50 Gew.-% eingebaute Polyethylenoxid-Einheiten enthalten können.

Als Polyesterpolyole geeignet sind z.B. Umsetzungsprodukte von mehrwertigen, vorzugsweise zweiwertigen und gegebenenfalls zusätzlich dreiwertigen Alkoholen mit mehrbasigen, vorzugsweise zweibasigen Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer aromatischer und/oder heterocyclischer Natur sein und gegebenenfalls z.B. durch Halogenatome substituiert und/oder ungesättigt sein. Beispielhaft sind genannt Adipinsäure, Phthalsäure, Isophthalsäure, Bemsteinsärue, Korksäure, Azelainsäure, Sebacinsäure, Trimellitsäure, Phthalsäureanhydrid, Tetrahydrophthalsäureanhydrid, Glutarsäureanhydrid, Tetrachlorphthalsäureanhydrid, Endomethylentetrahydrophthalsäureanhydrid, Maleinsäureanhydrid, Maleinsäure, Fumarsäure, dimere und trimere Fettsäuren wie Ölsäure, gegebenenfalls in Mischung mit monomeren Fettsäuren, Terephthalsäuredimethylester oder Terephthalsäure-bis-glykolester.

Bevorzugt sind unterhalb 60°C schmelzende Hydroxypolyester mit 2 oder 3 endständigen OH-Gruppen.

Die in Frage kommenden Polycarbonatpolyole sind durch Reaktion von Kohlensäurederivaten, z.B. Diphenylcarbonat, Dimethylcarbonat oder Phosgen, mit Diolen erhältlich. Als derartige Diole kommen z.B. Ethylenglykol, Triethylenglykol, Tetraethylenglykol, Propandiol-1,2 und -1,3, Butandiol-1,4 und -1,3, Pentandiol-1,5, Hexandiol-1,6, Octandiol-1,8, Neopentylgkylol, 1,4-Bis(hydroxymethyl)cyclohexan, Bis(hydroxymethyl)tricyclo[5.2.1.0^{2.6}] decan oder 1,4-Bis(2-hydroxyethoxy)benzol, 2-Methyl-1,3-propandiol, 2,2,4-Trimethylpentandiol, Dipropylenglykol, Polypropylenglykole, Dibutylenglykol, Polybutylenglykole, Bisphenol A und Tetrabrombisphenol A oder Mischungen der genannten Diole in Frage. Bevorzugt erhält die Diolkomponente 40 bis 100 Gew.% Hexandiol, vorzugsweise Hexandiol-1,6, und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol Caprolacton gemäß DE-A 1 770 245, oder durch Veretherung von Hexandiol mit sich selbst zum Di- oder Trihexylenglykol erhalten wurden. Die Herstellung solcher Derivate ist z.B. aus der DE-A 1 570 540 bekannt. Auch die in der DE-A 3 717 060 beschriebenen Polyether-Polycarbonatdiole können sehr gut eingesetzt werden.

Die Hydroxypolycarbonate sollen im wesentlichen linear sein. Sie können aber auch gegebenenfalls durch den Einbau polyfunktioneller Komponenten, insbesondere niedermolekularer Polyole, leicht verzweigt werden. Hierzu eignen sich beispielsweise Trimethylolpropan, Hexantriol-1,2,6, Glycerin, Butantriol-1,2,4, Pentaerythrit, Chinit, Mannit, Sorbit, Methylglykosid und 4,3,6-Dianhydrohexite.

Weiterhin können hydrophilierend wirkende Gruppen eingebaut werden, insbesondere wenn eine Verwendung aus wässrigem Medium, z.B. in einem wässrigen Lack, vorgesehen ist. Hydrophilierend wirkende Gruppen sind ionische Gruppen, die entweder kationischer oder anionischer Natur sein können und/oder nichtionische hydrophile Gruppen. Kationisch, anionisch oder nichtionisch dispergierend wirkende Verbindungen sind solche, die beispielsweise Sulfonium-, Ammonium-, Phosphonium-, Carboxylat-, Sulfonat-, Phosphonat-Gruppen oder die Gruppen, die durch Salzbildung in die vorgenannten Gruppen überführt werden können (potentiell ionische Gruppen) oder Polyethergruppen enthalten und durch vorhandene isocyanatreaktive Gruppen eingebaut werden können. Bevorzugt geeignete isocyanatreaktive Gruppen sind Hydroxyl- und Amingruppen.

Geeignete ionische oder potentiell ionische Gruppen enthaltende Verbindungen sind z.B. Mono- und Dihydroxycarbonsäuren, Mono- und Diaminocarbonsäuren, Mono- und Dihydroxysulfonsäuren, Mono- und Diaminosulfonsäuren sowie Mono- und Dihydroxyphosphonsäuren oder Mono- und Diaminophosphonsäuren und ihre Salze wie Dimethylolpropionsäure, Dimethylolbuttersäure, Hydroxypivalinsäure, N-(2-Aminoethyl)-β-alanin, 2-(2-Amino-ethylamino)-ethansulfonsäure, Ethylendiamin-propyl- oder butylsulfonsäure, 1,2- oder 1,3-Propylendiamin-β-ethylsulfonsäure, Äpfelsäure, Zitronensäure, Glykolsäure, Milchsäure, Glycin, Alanin, Taurin, Lysin, 3,5-Diaminobenzoesäure, ein Additionsprodukt von IPDI und Acrylsäure (EP-A 0 916 647, Beispiel 1) und dessen Alkali- und/oder Ammoniumsalze; das Addukt von Natriumbisulfit an Buten-2-diol-1,4, Polyethersulfonat, das propoxylierte Addukt aus 2-Butendiol und NaHSO₃, z.B. beschrieben in der DE-A 2 446 440 (Seite 5-9, Formel I-III) sowie in kationische Gruppen überführbare Bausteine wie N-Methyl-diethanolamin als hydrophile Aufbaukomponenten. Bevorzugte ionische oder potentielle ionische Verbindungen sind solche, die über Carboxy- oder Carboxylat- und/oder Sulfonatgruppen und/oder Ammoniumgruppen verfügen. Besonders bevorzugte ionische Verbindungen sind solche, die Carboxyl- und/oder Sulfonatgruppen als ionische oder potentiell ionische Gruppen enthalten, wie die Salze von N-(2-Aminoethyl)-β-alanin, der 2-(2-Aminoethylamino-)ethansulfonsäure oder des Additionsproduktes von IPDI und Acrylsäure (EP-A 0 916 647, Beispiel 1) sowie der Dimethylolpropionsäure.

Geeignete nichtionisch hydrophilierend wirkende Verbindungen sind z.B. Polyoxyalkylenether, die mindestens eine Hydroxy- oder Aminogruppe enthalten. Diese Polyether enthalten einen Anteil von 30 Gew.-% bis 100 Gew.-% an Bausteinen, die vom Ethylenoxid abgeleitet sind. In Frage kommen linear aufgebaute Polyether einer Funktionalität zwischen 1 und 3, aber auch Verbindungen der allgemeinen Formel (I), 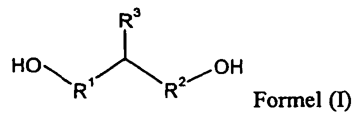 in welcher
- R¹ und R²: unabhängig voneinander jeweils einen zweiwertigen aliphatischen, cycloaliphatischen oder aromatischen Rest mit 1 bis 18 C-Atomen, die durch Sauerstoff und/oder Stickstoffatome unterbrochen sein können, bedeuten und
- R³: für einen alkoxyterminierten Polyethylenoxidrest steht.

Nichtionisch hydrophilierend wirkende Verbindungen sind beispielsweise auch einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4.Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bei den Polyalkylenoxidpolyetheralkoholen handelt es sich entweder um reine Polyethylenoxidpolyether oder gemischte Polyalkylenoxidpolyether, deren Alkylenoxideinheiten zu mindestens 30 mol-% bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten aufweisen.

Insbesondere bei der Verwendung eines ionische Gruppen enthaltenden Hydrophilierungsmittel muss sein Einfluss auf die Wirkung der Katalysatoren D) und vor allem E) überprüft werden. Aus diesem Grund sind nichtionische Hydrophilierungsmittel bevorzugt.

Als Verbindungen der Katalysatorkomponente D) kommen dem Fachman an sich bekannte Urethanisierungskatalysatoren wie Organozinnverbindungen oder aminische Katalysatoren in Frage. Als Organozinnverbindungen seien beispielhaft genannt: Dibutylzinndiacetat, Dibutylzinndilaurat, Dibutylzinn-bis-acetoacetonat und Zinncarboxylate wie beispielsweise Zinnoctoat. Die genannten Zinnkatalysatoren können gegebenenfalls in Kombination mit aminischen Katalysatoren wie Aminosilanen oder 1,4-Diazabicyclo[2.2.2]octan verwendet werden.

Besonders bevorzugt wird in D) Dibutylzinndilaurat als Urethanisierungskatalysator eingesetzt.

Im erfindungsgemäßen Verfahren wird die Katalysatorkomponente D) sofern überhaupt mitverwendet in Mengen von 0,001 bis 5,0 Gew.-%, bevorzugt 0,001 bis 0,1 Gew.% und besonders bevorzugt 0,005 - bis 0,05 Gew.-% bezogen auf Festgehalt des Verfahrensprodukts eingesetzt.

Als Katalysator E) können dem Fachmann an sich bekannte Allophanatisierungskatalysatoren, wie die Zinksalze Zinkoctoat, Zinkacetylacetonat und Zink-2-ethylcaproat, oder Tetraalkylammoniumverbindungen, wie *N*,*N*,*N-*Trimethyl-*N*-2-hydroxypropylammoniumhydroxid, *N*,*N*,*N-*Trimethyl-*N-*2-hydroxypropylammonium-2-ethylhexanoat oder Cholin-2-ethylhexanoat verwendet werden. Bevorzugt ist die Verwendung der Tetraalkylammoniumverbindungen, besonders bevorzugt diejenige von Tetraalkylammoniumalkanoaten und ganz besonders bevorzugt ist die von Cholin-2-ethylhexanoat als Allophanatisierungskatalysator.

Der Allophanatisierungskatalysator wird in Mengen von 0,001 - 5,0 Gew.-%, bevorzugt 0,01 - 1,0 Gew.-% und besonders bevorzugt 0,05 - 0,5 Gew.-% bezogen auf Festgehalt des Verfahrensprodukts eingesetzt.

Grundsätzlich kann schon für die Urethanisierungsreaktion in D) der Allophanatisierungskatalysator E) verwendet werden und die zweistufige Verfahrensweise zu einer einstufigen Umsetzung vereinfacht werden. Dies ist aber nicht bevorzugt, so dass der Allophanatisierungskatalysator erst dann zugesetzt wird, wenn die Urethangruppen ganz oder anteilig zu Allophanatgruppen umgesetzt werden sollen.

Der Katalysator E) kann in einer Portion auf einmal oder aber auch portionsweise oder auch kontinuierlich zugegeben werden. Bevorzugt ist eine portionsweise oder kontinuierliche Zugabe, um Temperaturspitzen und dadurch induzierte unerwünschte Polymerisationsreaktionen der strahlenhärtenden Gruppen zu vermeiden. Besonders bevorzugt wird der Katalysator E) in einer Geschwindigkeit von 200 - 600 ppm/h zugegeben und der Reaktionsansatz zur Vervollständigung der Allophanatisierung solange weitergerührt, bis der gewünschte NCO-Gehalt des Endproduktes erreicht ist.

Bevorzugt wird die Reaktion der Allophanatisierung solange geführt, bis der NCO-Gehalt des Produktes unter 0,5 Gew.%, besonders bevorzugt unter 0,1 Gew.% liegt.

Es ist grundsätzlich möglich, einen Restgehalt an NCO-Gruppen mit NCO-reaktiven Verbindungen wie z.B. Alkoholen nach beendeter Allophanatisierungsreaktion umzusetzen. Dadurch werden Produkte mit ganz besonders niedrigen NCO-Gehalten erhalten.

Es ist auch möglich die Katalysatoren D) und/oder E) nach dem Fachmann bekannten Methoden auf Trägermaterialien zu bringen und als heterogene Katalysatoren zu verwenden.

Im erfindungsgemäßen Verfahren können optional an beliebiger Stelle Lösemittel oder Reaktivverdünner eingesetzt werden.

Geeignete Lösemittel sind gegenüber den vorhandenen funktionellen Gruppen des Verfahrensprodukts vom Zeitpunkt der Zugabe bis zum Ende des Verfahrens inert. Geeignet sind z.B. in der Lacktechnik verwendete Lösemittel wie Kohlenwasserstoffe, Ketone und Ester, z.B. Toluol, Xylol, Isooctan, Aceton, Butanon, Methylisobutylketon, Ethylacetat, Butylacetat, Tetrahydrofuran, N-Methylpyrrolidon, Dimethylacetamid, Dimethylformamid, wobei jedoch bevorzugt kein Lösungsmittel zugesetzt wird.

Als Reaktivverdünner können Verbindungen mitverwendet werden, die bei der UV-Härtung ebenfalls (co)polymerisieren und somit in das Polymernetzwerk mit einbauen und gegenüber NCO-Gruppen inert sind. Solche Reaktivverdünner sind in P. K. T. Oldring (Ed.), Chemistry & Technology of UV & EB Formulations For Coatings, Inks & Paints, Vol. 2, 1991, SITA Technology, London, S. 237 - 285 exemplarisch beschrieben. Dies können Ester der Acrylsäure oder Methacrylsäure, bevorzugt der Acrylsäure mit mono- oder mehrfachfunktionellen Alkoholen sein. Als Alkohole eignen sich beispielsweise die isomeren Butanole, Pentanole, Hexanole, Heptanole, Octanole, Nonanole und Decanole, weiterhin cycloaliphatische Alkohole wie Isobornol, Cyclohexanol und alkylierte Cyclohexanole, Dicyclopentanol, arylaliphatischer Alkohole wie Phenoxyethanol und Nonylphenylethanol, sowie Tetrahydrofurfurylalkohole. Weiterhin können alkoxylierte Derivate dieser Alkohole verwendet werden. Geeignete zweiwertige Alkohole sind beispielsweise Alkohole wie Ethylenglykol, Propandiol-1,2, Propandiol-1,3, Diethylenglykol, Dipropylenglykol, die isomeren Butandiole, Neopentylglykol, Hexandiol-1,6, 2-Ethylhexandiol und Tripropylenglykol oder auch alkoxylierte Derivate dieser Alkohole. Bevorzugte zweiwertige Alkohole sind Hexandiol-1,6, Dipropylenglykol und Tripropylenglykol. Geeignete dreiwertige Alkohole sind Glycerin oder Trimethylolpropan oder deren alkoxylierte Derivate. Vierwertige Alkohole sind Pentaerythrit oder dessen alkoxylierte Derivate.

Die erfindungsgemäßen Bindemittel müssen gegen vorzeitige Polymerisation stabilisiert werden. Daher gibt man als Bestandteil von Komponente A) oder B) vor und/oder während der Reaktion Stabilisatoren zu, die die Polymerisation inhibieren. Bevorzugt ist dabei die Verwendung von Phenothiazin. Mögliche andere Stabilisatoren sind Phenole wie para-Methoxyphenol, 2,5-Di-tert.-Butylhydrochinon oder 2,6-Di-tert.-butyl-4-methylphenol. Geeignet sind auch N-Oxyl-Verbindungen zur Stabilisierung wie z.B. 2,2,6,6-Tetramethylpiperidin-N-Oxid (TEMPO) oder seine Derivate. Ebenso können die Stabilisatoren auch chemisch mit in das Bindemittel eingebaut werden, dabei eignen sich Verbindungen der oben genannten Klassen insbesondere wenn sie noch weitere freie aliphatische Alkoholgruppen oder primäre oder sekundäre Amingruppen tragen und damit über Urethan- oder Harnstoff Gruppen chemisch an Verbindungen der Komponente A) gebunden werden können. Hierfür besonders geeignet ist 2,2,6,6-Tetramethyl-4-hydroxy-piperidin-N-Oxid.

Andere Stabilisatoren wie z. B. Verbindungen der Klasse der HALS (HALS = hindered amine light stabilizers) werden dagegen weniger bevorzugt in E) eingesetzt, da sie bekanntermaßen keine so effektive Stabilisierung zu ermöglichen und vielmehr zu einer "schleichenden" radikalischen Polymerisation ungesättigter Gruppen führen können.

Die Stabilisatoren sind so zu wählen, dass sie unter dem Einfluss der Katalysatoren D) und E) stabil sind und unter den Reaktionsbedingungen nicht mit einer Komponente des erfindungsgemäßen Verfahrens reagieren. Dies kann zum Verlust der Stabilisierungseigenschaft führen.

Zur Stabilisierung der Reaktionsmischung, insbesondere der ungesättigten Gruppen gegen vorzeitige Polymerisation, kann ein sauerstoffhaltiges Gas, bevorzugt Luft, in und/oder über das Reaktionsgemisch geleitet werden. Es ist bevorzugt, dass das Gas einen möglichst geringen Anteil an Feuchtigkeit besitzt, um unerwünschte Reaktion bei Vorhandensein von Isocyanat zu verhindern.

In der Regel wird während der Herstellung der erfindungsgemäßen Bindemittel ein Stabilisator zugesetzt und abschließend, um eine Langzeitstabilität zu erreichen, nochmals mit einem phenolischen Stabilisator nachstabilisiert und ggf. das Reaktionsprodukt mit Luft gesättigt.

Im erfindungsgemäßen Verfahren wird die Stabilisatorkomponente typischerweise in Mengen von 0,001 bis 5,0 Gew.-%, bevorzugt 0,01 bis 2,0 Gew.-% und besonders bevorzugt 0,05 bis 1,0 Gew.-% bezogen auf Festgehalt des Verfahrensprodukts eingesetzt.

Das erfindungsgemäße Verfahren wird bei Temperaturen von höchstens 100°C, bevorzugt 20 bis 100°C, besonders bevorzugt von 40 bis 100°C, insbesondere bei 60 bis 90 °C durchgeführt.

Es ist unerheblich, ob eine oder beide Stufen des erfindungsgemäßen Verfahrens kontinuierlich z.B. in einem Statik-Mischer, Extruder oder Kneter oder diskontinuierlich z.B. in einem Rührreaktor durchgeführt werden.

Bevorzugt wird das erfindungsgemäße Verfahren in einem Rührreaktor durchgeführt.

Der Verlauf der Reaktion kann durch geeignete im Reaktionsgefäß installierte Messgeräte und/oder anhand von Analysen an entnommenen Proben verfolgt werden. Geeignete Verfahren sind dem Fachmann bekannt. Es handelt sich beispielsweise um Viskositätsmessungen, Messungen des NCO-Gehalts, des Brechungsindex, des OH-Gehalts, Gaschromatographie (GC), kernmagnetische Resonanzspektroskopie (NMR), Infrarotspektroskopie (IR) und nahe Nahinfrarotspektroskopie (NIR). Bevorzugt ist die IR-Kontrolle auf vorhandene freie NCO Gruppen (für aliphatische NCO-Gruppen, Bande bei ca. ν = 2272 cm⁻¹) und GC-Untersuchungen auf nicht umgesetzte Verbindungen aus A), B) und ggf. C).

Es ist grundsätzlich möglich, das erfindungsgemäße Verfahren in einer Stufe durchzuführen, wobei mit einem Katalysator oder einem Katalysatorgemisch, das sowohl die Urethanisierungs- als auch die Allophanatisierungsreaktion katalysiert, gearbeitet wird. Dabei werden Urethanisierung und Allophanatisierung parallel durchgeführt. Diese Verfahrensweise ist aber nicht bevorzugt.

Die nach dem erfindungsgemäßen Verfahren erhältlichen ungesättigten Allophanate, insbesondere die auf Basis des bevorzugt eingesetzten HDI, weisen bevorzugt Scherviskositäten bei 23°C von ≤ 150 000 mPas, besonders bevorzugt ≤ 80 000 mPas auf.

Die nach dem erfindungsgemäßen Verfahren erhältlichen ungesättigten Allophanate, insbesondere die auf Basis des bevorzugt eingesetzten HDI, weisen bevorzugt zahlenmittlere Molekulargewichte Mₙ von 600 bis 3000 g/mol, besonders bevorzugt 650 bis 1500 g/mol auf.

Die nach dem erfindungsgemäßen Verfahren erhältlichen ungesättigten Allophanate weisen bevorzugt Gehalte an freien Di- bzw. Triisocyanatmonomeren von unter 0,5 Gew.-%, besonders bevorzugt unter 0,1 Gew.% auf.

Die erfindungsgemäßen strahlenhärtenden Allophanate können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden. Im Falle der Verklebung oder Abdichtung ist allerdings Voraussetzung, dass bei UV-Stahlenhärtung mindestens eines der beiden zu verklebenden oder miteinander abzudichtenden Substrate für UV-Strahlung durchlässig, also i. d. R. transparent sein muss. Bei der Elektronenstrahlung ist auf eine hinreichende Durchlässigkeit für Elektronen zu achten. Bevorzugt ist die Verwendung in Lacken und Beschichtungen.

Ein weiterer Gegenstand der Erfindung sind Beschichtungsmittel enthaltend
a) ein oder mehrere der erfindungsgemäßen strahlenhärtenden Allophanate,
b) gegebenenfalls ein oder mehrere Polyisocyanate mit freien oder blockierten Isocyanatgruppen, die frei sind von unter Einwirkung aktinischer Strahlung mit ethylenisch ungesättigten Verbindungen unter Polymerisation reagierenden Gruppen,
c) gegebenenfalls weitere von denen aus a) verschiedene Verbindungen, die unter Einwirkung aktinischer Strahlung mit ethylenisch ungesättigten Verbindungen unter Polymerisation reagierende Gruppen und gegebenenfalls freie oder blockierte NCO-Gruppen aufweisen,
d) gegebenenfalls ein oder mehrere mit Isocyanaten reagierende, aktiven Wasserstoff enthaltende Verbindungen,
e) Initiatoren,
f) gegebenenfalls Lösemittel und
g) gegebenenfalls Hilfs- und Zusatzstoffe.

Die Polyisocyanate der Komponente b) sind dem Fachmann an sich bekannt. Bevorzugt werden hier gegebenenfalls mit Isocyanurat-, Allophanat-, Biuret-, Uretdion- und/oder Iminooxadiazintriongruppen modifizierte Verbindungen auf Hexamethylendiisocyanat-, Isophorondiisocyanat-, 4,4'-Diisocyanatodicyclohexylmethan- und/oder Trimethylhexamethylendiisocyanat-Basis verwendet.

Die NCO-Gruppen können dabei auch blockiert sein, wobei als Blockierungsmittel die bereits bei Beschreibung der Komponente A) genannten Verbindungen zum Einsatz kommen.

Zu den Verbindungen der Komponente c) gehören Verbindungen wie insbesondere Urethanacrylate bevorzugt auf Hexamethylendiisocyanat-, Isophorondiisocyanat-, 4,4'-Diisocyanatodicyclohexylmethan- und/oder Trimethylhexamethylendiisocyanat-Basis, welche gegebenenfalls mit Isocyanurat-, Allophanat-, Biuret-, Uretdion- und/oder Iminooxadiazintriongruppen modifiziert sein können, welche keine gegenüber Isocyanatgruppen reaktive, aktiven Wasserstoff enthaltene Funktionen aufweisen.

NCO-haltige Urethanacrylate sind kommerziell bei der Bayer AG, Leverkusen, DE als Roskydal® UA VP LS 2337, Roskydal® UA VP LS 2396 oder Roskydal® UA XP 2510 erhältlich.

Weiterhin können die bereits beschriebenen und in der Technik der strahlenhärtenden Beschichtungen bekannten Reaktivverdünner als Bestandteil von c) verwendet werden, sofern sie keine mit NCO-Gruppen reaktiven Gruppen enthalten.

Verbindungen der Komponente d) können gesättigt oder ungesättigt sein. Mit NCO-Gruppen reagierende chemische Funktionalitäten sind aktivierte Wasserstoffatome enthaltende Funktionalitäten wie Hydroyl, Amin oder Thiol.

Bevorzugt sind gesättigte Polyhydroxyverbindungen , z.B. die an sich aus der Technolgie des Beschichtens, des Klebens, der Druckfarben oder der Dichtungsmassen bekannten Polyetherpolyole, Polyesterpolyole, Polycarbonatpolyole, Poly(meth)acrylatpolyole, Polyurethanpolyole, die keine unter Einwirkung aktinischer Strahlung mit ethylenisch ungesättigten Verbindungen unter Polymerisation reagierende Gruppen aufweisen.

Ungesättigte hydroxyfunktionelle Verbindungen sind z.B. die in der Technik der strahlenhärtenden Beschichtungen bekannten Epoxyacrylate, Polyesteracrylate, Polyetheracrylate, Urethanacrylate sowie acrylierte Polyacrylate, die eine OH-Zahl von 30 bis 300 mg KOH/g aufweisen.

Weiterhin können die bereits beschriebenen und in der Technik der strahlenhärtenden Beschichtungen bekannten Reaktivverdünner als Bestandteil von d) verwendet werden, sofern sie mit NCO-Gruppen reaktive Gruppen enthalten.

Als Initiatoren der Komponente e) für eine radikalische Polymerisation können durch Strahlung und/oder thermisch aktivierbare Initiatoren zum Einsatz kommen. Fotoinitiatoren, die durch UV- oder sichtbares Licht aktiviert werden, sind hierbei bevorzugt. Fotoinitiatoren sind an sich bekannte, kommerziell vertriebene Verbindungen, wobei zwischen unimolekularen (Typ I) und bimolekularen (Typ II) Initiatoren unterschieden wird. Geeignete (Typ I)-Systeme sind aromatische Ketonverbindungen, z.B. Benzophenone in Kombination mit tertiären Aminen, Alkylbenzophenone, 4,4'-Bis(dimethylamino)benzophehon (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacylphosphinoxide, Phenylglyoxylsäureester, Campherchinon, α-Aminoalkylphenone, α,α-Dialkoxyacetophenone und α,-Hydroxyalkylphenone.

Die Initiatoren, die in Mengen zwischen 0,1 und 10 Gew.-%, vorzugsweise 0,1 bis 5 Gew.-%, bezogen auf das Gewicht des Lackbindemittels, eingesetzt werden, können als einzelne Substanz oder, wegen häufiger vorteilhafter synergistischer Effekte, auch in Kombination miteinander verwendet werden.

Werden Elektronenstrahlen statt UV-Strahlung verwendet, so benötigt man keinen Photoinitiator. Elektronenstrahlung wird wie dem Fachmann bekannt ist, mittels thermischer Emission erzeugt und über eine Potentialdifferenz beschleunigt. Die energiereichen Elektronen schlagen dann durch eine Titanfolie und werden auf die zu härtenden Bindemittel gelenkt. Die allgemeinen Prinzipien der Elektronenstrahlhärtung sind in "Chemistry & Technology of UV & EB Formulations for Coatings, Inks & Paints", Vol. 1, P K T Oldring (Ed.), SITA Technology, London, England, S. 101-157, 1991 im Detail beschrieben.

Im Falle einer thermischen Härtung der aktivierten Doppelbindungen, kann diese auch unter Zusatz von thermisch zerfallenden Radikalbildnern erfolgen. Geeignet sind, wie dem Fachmann bekannt ist, z.B. Peroxyverbindungen wie Dialkoxydicarbonate wie z.B. Bis(4-tert-butylcyclohexyl)peroxydicarbonat, Dialkylperoxide wie z.B. Dilaurylperoxid, Perester aromatischer oder aliphatischer Säuren wie z.B. tert.-Butylperbenzoat oder tert.-Amylperoxy 2-ethylhexanoat, anorganische Peroxide wie z. B. Ammoniumperoxodisulfat, Kaliumperoxodisulfat, organische Peroxide wie z.B. 2,2-Bis(tert.-butylperoxy)butan, Dicumylperoxid, tert.-Butylhydroperoxid oder auch Azoverbindungen wie 2,2'-Azobis[N-(2-propenyl)-2-methylpropionamide], 1-[(cyano-1-methylethyl)azo]formamide, 2,2'-Azobis(N-butyl-2-methylpropionamide), 2,2'-Azobis(N-cyclohexyl-2-methylpropionamide), 2,2'-Azobis{2-methyl-N-[2-(1-hydroxybutyl)]propionamide}, 2,2'-Azobis-{2-methyl-N-[2-(1-hydroxybutyl)]propionamide, 2,2'-Azobis {2-methyl-N-[1,1-bis(hydroxymethyl)-2-hydroxyethyl] propionamide. Möglich sind auch hochsubstituierte 1,2-Diphenylethane (Benzpinakole), wie z. B. 3,4-Dimethyl-3,4-diphenylhexan, 1,1,2,2-Tetraphenyl-ethandiol-1,2 oder auch deren silylierten Derivate.

Es ist auch möglich eine Kombination von durch UV-Licht und thermisch aktivierbaren Initiatoren zu verwenden.

Zu den Hilfs- und Zusatzstoffen der Komponente e) gehören Lösemittel der vorstehend genannten Art.

Des Weiteren können in e) zur Erhöhung der Wetterstabilität der gehärteten Lackschicht auch UV-Absorber und/oder HALS-Stabilisatoren enthalten sein. Bevorzugt ist die Kombination. Erstere sollten einen Absorptionsbereich von maximal 390 nm haben wie Triphenyltriazintypen (z.B. Tinuvin® 400 (Ciba Spezialitätenchemie GmbH, Lampertheim, DE)), Benztriazole wie Tinuvin® 622 (Ciba Spezialitätenchemie GmbH, Lampertheim, DE) oder Oxalsäuredianilide (z. B. Sanduvor® 3206 (Clariant, Muttenz, CH))) und werden in 0,5 bis 3,5 Gew.-% bezogen auf Festharz zugegeben. Geeignete HALS-Stabilisatoren sind kommerziell erhältlich (Tinuvin® 292 oder Tinuvin® 123 (Ciba Spezialitätenchemie GmbH, Lampertheim, DE) oder Sanduvor® 3258 (Clariant, Muttenz, CH). Bevorzugte Mengen sind 0,5-2,5 Gew.-% bezogen auf Festharz.

Ebenfalls können in e) Pigmente, Farbstoffe, Füllstoffe, Verlaufs- und Entlüftungsadditive enthalten sein.

Darüber hinaus können, sofern erforderlich, die aus der Polyurethanchemie bekannten Katalysatoren zur Beschleunigung der NCO/OH-Reaktion in e) enthalten sein. Dies sind z.B. Zinn- oder Zinksalze oder Zinnorganoverbindungen, Zinn- und/oder Zinkseifen wie z.B. Zinnoctoat, Dibutylzinndilaurat, Dibutylzinnoxid oder tertiäre Amine wie z. B. Diazabicyclo[2,2,2]octan (DABCO).

Das Auftragen der erfindungsgemäßen Beschichtungsmittel auf das zu beschichtende Material erfolgt mit den in der Beschichtungstechnologie üblichen und bekannten Methoden wie Spritzen, Rakeln, Walzen, Gießen, Tauchen, Schleudern, Streichen oder Sprühen oder durch Druck-Techniken wie Sieb-, Tief-, Flexo- oder Offsetdruck sowie durch Transfer-Methoden.

Geeignete Substrate sind beispielsweise Holz, Metall, insbesondere auch Metall wie es in den Anwendungen der sogenannten Draht-, Coil-, Can- oder Container-Lackierung verwendet wird, weiterhin Kunststoff auch in Form von Folien, insbesondere ABS, AMMA, ASA, CA, CAB, EP, UF, CF, MF, MPF, PF, PAN, PA, PE, HDPE, LDPE, LLDPE, UHMWPE, PET, PMMA, PP, PS, SB, PUR, PVC, RF, SAN, PBT, PPE, POM, PUR-RIM, SMC, BMC, PP-EPDM, und UP (Kurzbezeichnungen nach DIN 7728T1), Papier, Leder, Textilien, Filz, Glas, Holz, Holzwerkstoffe, Kork, anorganisch gebundene Substrate wie Holz- und Faserzementplatten, elektronische Baugruppen oder mineralische Untergründe. Es können auch Substrate, die aus verschiedenen der vorgenannten Materialien bestehen, oder bereits beschichtete Substrate wie Fahrzeuge, Flugzeuge oder Schiffe sowie deren Teile, insbesondere Karosserien oder Anbauteile lackiert werden. Es ist auch möglich die Beschichtungsmittel nur temporär auf ein Substrat aufzubringen, dann teilweise oder vollständig zu härten und gegebenenfalls wieder abzulösen, um z.B. Folien herzustellen.

Zur Aushärtung können durch Ablüften z.B. enthaltene Lösemittel ganz oder teilweise entfernt werden.

Anschließend oder gleichzeitig können der oder die gegebenenfalls notwendigen thermischen und der und die photochemischen Aushärtungsprozesse nacheinander oder gleichzeitig durchgeführt werden.

Falls notwendig kann die thermische Härtung bei Raumtemperatur aber auch bei erhöhter Temperatur, vorzugsweise bei 40 bis 160°C, bevorzugt bei 60 bis 130°C, besonders bevorzugt bei 80 bis 110°C erfolgen.

Bei Verwendung von Photoinitiatoren in d) erfolgt die Strahlungshärtung bevorzugt durch Einwirkung energiereicher Strahlung, also UV-Strahlung oder Tageslicht, z.B. Licht der Wellenlänge 200 bis 700 nm oder durch Bestrahlen mit energiereichen Elektronen (Elektronenstrahlung, 150 bis 300 keV). Als Strahlungsquellen für Licht oder UV-Licht dienen beispielsweise Hoch- oder Mitteldruckquecksilberdampflampen, wobei der Quecksilberdampf durch Dotierung mit anderen Elementen wie Gallium oder Eisen modifiziert sein kann. Laser, gepulste Lampen (unter der Bezeichnung UV-Blitzlichtstrahler bekannt), Halogenlampen oder Eximerstrahler sind ebenfalls möglich. Die Strahler können bauartbedingt oder durch Einsatz spezieller Filter und/oder Reflektoren so ausgestattet sein, das der Austritt eines Teils des UV-Spektrums verhindert wird. Beispielsweise kann z.B. aus arbeitshygienischen Gründen die dem UV-C oder UV-C und UV-B zugeordnete Strahlung herausgefiltert werden. Die Strahler können ortsunbeweglich installiert sein, so dass das zu bestrahlende Gut mittels einer mechanischen Vorrichtung an der Strahlungsquelle vorbeibewegt wird oder die Strahler können beweglich sein und das zu bestrahlende Gut verändert bei der Härtung seinen Ort nicht. Die üblicherweise zur Vernetzung ausreichende Strahlungsdosis bei UV-Härtung liegt im Bereich von 80 bis 5000 mJ/cm².

Die Bestrahlung kann gegebenenfalls auch unter Ausschluss von Sauerstoff, z. B. unter InertgasAtmosphäre oder Sauerstoff-reduzierter Atmosphäre durchgeführt werden. Als Inertgase eignen sich bevorzugt Stickstoff, Kohlendioxid, Edelgase oder Verbrennungsgase. Des Weiteren kann die Bestrahlung erfolgen, indem die Beschichtung mit für die Strahlung transparenten Medien abgedeckt wird. Beispiele hierfür sind z.B. Kunststofffolien, Glas oder Flüssigkeiten wie Wasser.

Je nach Strahlungsdosis und Aushärtungsbedingungen sind Typ und Konzentration des gegebenenfalls verwendeten Initiators in dem Fachmann bekannter Weise zu variieren.

Besonders bevorzugt werden zur Härtung Quecksilberhochdruckstrahler in ortsfesten Anlagen eingesetzt. Fotoinitiatoren werden dann in Konzentrationen von 0,1 bis 10 Gew.%, besonders bevorzugt 0,2 bis 3,0 Gew.-% bezogen auf den Festkörper der Beschichtung eingesetzt. Zur Härtung dieser Beschichtungen wird bevorzugt eine Dosis von 200 bis 3000 mJ/cm² gemessen im Wellenlängebereich von 200 bis 600 nm verwendet.

Bei Verwendung von thermisch aktivierbaren Initiatoren in d) kann die Härtung durch Erhöhung der Temperatur ausgelöst werden. Die thermische Energie kann dabei durch Strahlung, Wärmeleitung und/oder Konvektion in die Beschichtung eingebracht werden, wobei üblicherweise die in der Beschichtungstechnologie gebräuchlichen Infrarot-Strahler, Nahinfrarotstrahler und/oder Öfen zum Einsatz kommen.

Die applizierten Schichtdicken (vor der Härtung) liegen typischerweise zwischen 0,5 und 5000 µm, bevorzugt zwischen 5 und 1000 µm, besonders bevorzugt zwischen 15 und 200 µm. Bei Verwendung von Lösungsmitteln werden diese nach der Applikation und vor der Härtung durch die gängigen Methoden entfernt.

### Beispiele

Alle Prozentangaben beziehen sich sofern nicht abweichend angegeben auf Gewichtsprozent.

Die Bestimmung der NCO-Gehalte in % wurde über Rücktitration mit 0,1 mol/l Salzsäure nach Reaktion mit Butylamin vorgenommen, Grundlage DIN EN ISO 11909.

Die Viskositätsmessungen wurden mit einem Platte-Platte Rotationsviskosimeter, RotoVisko 1 der Firma Haake, DE nach ISO/DIS 3219:1990 durchgeführt.

Die zur Zeit der Versuchsdurchführung herrschende Umgebungstemperatur von 23°C wird als RT bezeichnet.

### Herstellung von Cholin-2-ethylhexanoat

In einem 1000ml Glaskolben mit Rührvorrichtung wurden bei RT 83g Natrium-2-ethylhexanoat in 600ml Methanol gelöst. Es wurden anschließend 69,8g Cholinchlorid portionsweise zugegeben, und die Mischung wurde weitere 10 Stunden bei Raumtemperatur gerührt. Der entstandene Niederschlag wurde abfiltriert und die Lösung am Rotationsverdampfer unter vermindertem Druck auf etwa ein Drittel eingeengt, bis sich erneut ein Niederschlag bildete. Es wurde mit etwa 400ml Aceton verdünnt, erneut filtriert und das Lösungsmittel unter vermindertem Druck wiederum abgezogen. Der verbleibende Rest wurde abermals in etwa 400ml Aceton aufgenommen, filtriert und das Lösungsmittel abgezogen. Es wurden 117g kristallisationsstabiles, flüssiges Produkt erhalten, das in dieser Form als Allophanatisierungskatalysator eingesetzt wurde.

### Beispiel 1: Erfindungsgemäßes allophanathaltiges Bindemittel (NCO/OH=1,33:1)

In einem 500ml-Vierhals-Glaskolben mit Rückflusskühler, beheizbarem Ölbad, mechanischem Rührer, Luftdurchleitung (l/h), Innenthermometer und Tropftrichter wurden 231,16g Hexamethylendiisocyanat (Desmodur® H, Bayer MaterialScience, Leverkusen) und 50mg Phenothiazin vorgelegt und auf 70°C erwärmt. Es wurden 25mg Dibutylzinndilaurat (Desmorapid Z, Bayer MaterialScience, Leverkusen) zugegeben und 268,01g Hydroxypropylacrylat so zugetropft, dass die Temperatur 80°C nicht überschritt. Es wurde nachgerührt, bis der theoretische NCO-Wert von 5,77% erreicht war. Anschließend wurde die Temperatur auf 80°C erhöht, und es wurden über 6 Stunden 0,75g Cholin-2-ethylhexanoat langsam zudosiert. Nach etwas mehr als der Hälfte der Zeit war eine deutliche Exothermie zu verzeichnen, die eine Kühlung des Ansatzes nötig machte. Die Dosierung wurde trotzdem zu Ende geführt, und es wurde noch zwei weitere Stunden nachgerührt. Es wurde ein farbloses Harz mit einem Rest-NCO-Gehalt von 0,1% und einer Viskosität von 75.400 mPas (23°C) erhalten.

### Beispiel 2: Erfindungsgemäßes allophanathaltiges Bindemittel (NCO/OH= 1,25:1)

In einem 500ml-Vierhals-Glaskolben mit Rückflusskühler, beheizbarem Ölbad, mechanischem Rührer, Luftdurchleitung (l/h), Innenthermometer und Tropftrichter wurden 223,18g Hexamethylendiisocyanat und 50mg Phenothiazin vorgelegt und auf 70°C erwärmt. Es wurden 25mg Dibutylzinndilaurat zugegeben und 276,00g Hydroxypropylacrylat so zugetropft, dass die Temperatur 80°C nicht überschritt. Es wurde nachgerührt, bis der theoretische NCO-Wert von 4,46% erreicht war. Anschließend wurden bei 70°C über 6 Stunden 0,75g Cholin-2-ethylhexanoat langsam zudosiert. Gegen Ende der Zeit war eine deutliche Exothermie zu verzeichnen, die eine Kühlung des Ansatzes nötig machte. Die Dosierung wurde trotzdem zu Ende geführt, und es wurde noch zwei weitere Stunden nachgerührt. Es wurde ein farbloses Harz mit einem Rest-NCO-Gehalt von 0,05% und einer Viskosität von 35.800mPas(23°C) erhalten.

### Beispiel 3: Erfindungsgemäßes allophanathaltiges Bindemittel (NCO/OH= 1,43:1)

In einem 500ml-Vierhals-Glaskolben mit Rückflusskühler, beheizbarem Ölbad, mechanischem Rührer, Luftdurchleitung (l/h), Innenthermometer und Tropftrichter wurden 239,74g Hexamethylendiisocyanat und 50mg Phenothiazin vorgelegt und auf 70°C erwärmt. Es wurden 25mg Dibutylzinndilaurat zugegeben und 259,43g Hydroxypropylacrylat so zugetropft, dass die Temperatur 80°C nicht überschritt. Es wurde nachgerührt, bis der theoretische NCO-Wert von 7,18% erreicht war. Anschließend wurden bei 70°C über 6 Stunden 0,75g Cholin-2-ethylhexanoat langsam zudosiert. Nach etwas mehr als der Hälfte der Zeit war eine deutliche Exothermie zu verzeichnen, die eine Kühlung des Ansatzes nötig machte. Die Dosierung wurde trotzdem zu Ende geführt, und es wurde noch eine weitere Stunde nachgerührt. Es wurde ein farbloses Harz mit einem Rest-NCO-Gehalt von 0,0% und einer Viskosität von 125.000mPas(23°C) erhalten.

### Vergleichsbeispiel zu Beispiel 1-3: Nicht erfindungsgemäßes allophanathaltiges Bindemittel (NCO/OH =1,6:1)

In einem 500ml-Vierhals-Glaskolben mit Rückflusskühler, beheizbarem Ölbad, mechanischem Rührer, Luftdurchleitung (l/h), Innenthermometer und Tropftrichter wurden 268,8g Hexamethylendiisocyanat und 50mg Phenothiazin vorgelegt und auf 70°C erwärmt. Es wurden 25mg Dibutylzinndilaurat zugegeben und 260,0g Hydroxypropylacrylat so zugetropft, dass die Temperatur 80°C nicht überschritt. Es wurde nachgerührt, bis der theoretische NCO-Wert von 9,53% erreicht war. Anschließend wurden bei 70°C über 6 Stunden 0,75g Cholin-2-ethylhexanoat langsam zudosiert. Gegen Ende der Zeit war eine deutliche Exothermie zu verzeichnen, die eine Kühlung des Ansatzes nötig machte. Die Dosierung wurde trotzdem zu Ende geführt, und es wurde noch zwei weitere Stunden nachgerührt. Es wurde ein farbloses Harz mit einem Rest-NCO-Gehalt von 0,05% und einer nur noch schwer messbaren Viskosität von etwa 650.000mPas(23°C) erhalten.

### Beispiel 4: Erfindungsgemäßes allophanathaltiges Bindemittel (Misch-Typ, NCO/OH= 1,33:1)

In einem 500ml-Vierhals-Glaskolben mit Rückflusskühler, beheizbarem Ölbad, mechanischem Rührer, Luftdurchleitung (l/h), Innenthermometer und Tropftrichter wurde eine Mischung aus 107,59g Hexamethylendiisocyanat und 142,02 Isophorondiisocyanat (Desmodur® I, Bayer MaterialScience, Leverkusen) mit 250mg Phenothiazin vorgelegt und auf 70°C erwärmt. Es wurden 50mg Dibutylzinndilaurat zugegeben und 249,49g Hydroxypropylacrylat so zugetropft, dass die Temperatur 80°C nicht überschritt. Es wurde nachgerührt, bis der theoretische NCO-Wert von 5,37% erreicht war. Anschließend wurde die Temperatur auf 80°C erhöht und über 4 Stunden 0,75g Cholin-2-ethylhexanoat langsam zudosiert. Nach etwa der Hälfte der Zeit war eine deutliche Exothermie zu verzeichnen, die eine Kühlung des Ansatzes nötig machte. Nach Beendigung der Dosierung wurde noch zwei weitere Stunden nachgerührt und mit 125g Hexandioldiacrylat (Laromer® HDDA, BASF AG, Ludwigshafen, DE) verdünnt. Es wurde ein gelbliches Harz mit einem Rest-NCO-Gehalt von 0,17% und einer Viskosität von 20.500mPas(23°C) erhalten.

### Beispiel 5: Lackformulierung und Lack

Ein Teil des Produkts aus Beispiel 1 wurde mit 3,0 % des Photoinitiators Darocur® 1173 (Photoinitiator, Handelsprodukt der Ciba Spezialitätenchemie GmbH, Lampertheim, DE) intensiv gemischt. Mittels eines Knochen-Rakels mit einem Spalt von 90 µm wurde die Mischung als dünner Film auf eine Glasplatte aufgezogen. Nach UV-Bestrahlung (Quecksilbermitteldruckstrahler, IST Metz GmbH, Nürtingen, DE, 750 mJ/cm²) wurde ein transparente, harte Beschichtung erhalten, die sich durch Stahlwolle (Typ 0/0/0) mit einer auf den Film gerichteten Kraft von 500 g in zehn Doppelhüben kaum verkratzen ließ.

## Patentansprüche

1. Verfahren zur Herstellung von strahlenhärtenden Allophanaten mit Restmonomergehalten von weniger als 0,5 Gew.-% und einem NCO-Gehalt von weniger als 1 Gew.%, bei dem aus
A) isocyanatgruppenhaltigen Verbindungen,
B) hydroxyfunktionellen Verbindungen, die unter Einwirkung aktinischer Strahlung mit ethylenisch ungesättigten Verbindungen unter Polymerisation reagierende Gruppen (strahlenhärtende Gruppen) aufweisen und
C) gegebenenfalls weitere Verbindungen mit NCO-reaktiven Gruppen
D) gegebenenfalls in Anwesenheit eines Katalysators NCO-gruppenhaltige Urethane mit strahlenhärtenden Gruppen gebildet werden, die anschließend ohne weitere Zugabe an isocyanatgruppenhaltigen Verbindungen in Anwesenheit
E) eines Allophanatisierungskatalysators umgesetzt werden,
wobei das Verhältnis von NCO-Gruppen der Verbindungen aus A) zu den OH-Gruppen von den Verbindungen aus B) und ggf. C) 1,45 : 1,0 bis 1,1 : 1,0 beträgt.

2. Verfahren zur Herstellung von strahlenhärtenden Allophanaten gemäß Anspruch 1, **dadurch gekennzeichnet, dass** in Komponente A) Hexamethylendiisocyanat (HDI), Isophorondiisocyanat (IPDI) und/oder 4,4'-Diisocyanatodicyclohexylmethan eingesetzt werden.

3. Verfahren zur Herstellung von strahlenhärtenden Allophanaten gemäß Anspruch 1 oder 2, **dadurch gekennzeichnet, dass** in Komponente B) Hydroxyethyl(meth)acrylat, Hydroxypropyl(meth)acrylat und/oder Hydroxybutyl(meth)acrylat eingesetzt werden.

4. Verfahren zur Herstellung von strahlenhärtenden Allophanaten gemäß einem der Ansprüche 1 bis 3, **dadurch gekennzeichnet, dass** das Verhältnis von NCO-Gruppen der Verbindungen aus A) zu den OH-Gruppen von den Verbindungen aus B) und ggf. C) 1,35 : 1,0 bis 1,3 : 1,0 beträgt.

5. Verfahren zur Herstellung von strahlenhärtenden Allophanaten gemäß einem der Ansprüche 1 bis 4, **dadurch gekennzeichnet, dass** Katalysator E) in einer Geschwindigkeit von 200 - 600 ppm/h zugegeben wird.

6. Verfahren zur Herstellung von strahlenhärtenden Allophanaten gemäß einem der Ansprüche 1 bis 5, **dadurch gekennzeichnet, dass** die Allophanatisierung solange geführt wird, bis das Endprodukt einen NCO-Gehalt von unter 0,1 Gew.-% aufweist.

7. Strahlenhärtende Allophanate erhältlich nach einem Verfahren gemäß einem der Ansprüche 1 bis 6.

8. Verwendung der strahlenhärtenden Allophanate gemäß Anspruch 7 bei der Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen.

9. Beschichtungsmittel enthaltend
a) ein oder mehrere der strahlenhärtenden Allophanate gemäß Anspruch 7,
b) gegebenenfalls ein oder mehrere Polyisocyanate mit freien oder blockierten Isocyanatgruppen, die frei sind von unter Einwirkung aktinischer Strahlung mit ethylenisch ungesättigten Verbindungen unter Polymerisation reagierenden Gruppen,
c) gegebenenfalls weitere von denen aus a) verschiedene Verbindungen, die unter Einwirkung aktinischer Strahlung mit ethylenisch ungesättigten Verbindungen unter Polymerisation reagierende Gruppen und gegebenenfalls freie oder blockierte NCO-Gruppen aufweisen,
d) gegebenenfalls ein oder mehrere mit Isocyanaten reagierende, aktiven Wasserstoff enthaltende Verbindungen,
e) Initiatoren,
f) gegebenenfalls Lösemittel und
g) gegebenenfalls Hilfs- und Zusatzstoffe.

10. Substrate beschichtet mit Beschichtungen erhältlich unter Verwendung von strahlenhärtenden Allophanate gemäß Anspruch 7.

## Claims

1. Process for preparing radiation-curing allophanates having residual monomer contents of less than 0.5% by weight and an NCO content of less than 1% by weight, wherein
A) compounds containing isocyanate groups,
B) hydroxy-functional compounds which contain groups which react, with polymerization, with ethylenically unsaturated compounds on exposure to actinic radiation (radiation-curing groups) and
C) optionally further compounds containing NCO-reactive groups
D) optionally in the presence of a catalyst are used to form NCO-group-containing urethanes having radiation-curing groups, which are subsequently reacted, without further addition of compounds containing isocyanate groups, in the presence
E) of an allophanatization catalyst,
the ratio of NCO groups of the compounds from A) to the OH groups of the compounds from B) and, where used, C) being 1.45 : 1.0 to 1.1 : 1.0.

2. Process for preparing radiation-curing allophanates according to Claim 1, **characterized in that** in component A) hexamethylene diisocyanate (HDI), isophorone diisocyanate (IPDI) and/or 4,4'-diisocyanatodicyclohexylmethane are used.

3. Process for preparing radiation-curing allophanates according to Claim 1 or 2, **characterized in that** in component B) hydroxyethyl (meth)acrylate, hydroxypropyl (meth)acrylate and/or hydroxybutyl (meth)acrylate are used.

4. Process for preparing radiation-curing allophanates according to any one of Claims I to 3, **characterized in that** the ratio of NCO groups of the compounds from A) to the OH groups of the compounds from B) and, where used, C) is 1.35 : 1.0 to 1.3 : 1.0.

5. Process for preparing radiation-curing allophanates according to any one of Claims 1 to 4, **characterized in that** catalyst E) is added at a rate of 200 - 600 ppm/h.

6. Process for preparing radiation-curing allophanates according to any one of Claims 1 to 5, **characterized in that** the allophanatization is carried out until the end product has an NCO content of below 0.1 % by weight.

7. Radiation-curing allophanates, obtainable by a process according to any one of Claims 1 to 6.

8. Use of the radiation-curing allophanates according to Claim 7 in the preparation of coatings, coating materials, adhesives, printing inks, casting resins, dental compounds, sizes, photoresists, stereolithography systems, resins for composite materials and sealants.

9. Coating compositions comprising
a) one or more of the radiation-curing allophanates according to Claim 7,
b) optionally one or more polyisocyanates containing free or blocked isocyanate groups, which are free from groups which react, with polymerization, with ethylenically unsaturated compounds on exposure to actinic radiation,
c) optionally other compounds, different from those of a), which contain groups which react, with polymerization, with ethylenically unsaturated compounds on exposure to actinic radiation, and optionally contain free or blocked NCO groups,
d) optionally one or more isocyanate-reactive compounds containing active hydrogen,
e) initiators,
f) optionally solvents and
g) optionally auxiliaries and additives.

10. Substrates coated with coatings obtainable using radiation-curing allophanates according to Claim 7.

## Revendications

1. Procédé pour la préparation d'allophanates durcissables par irradiation, ayant des teneurs en monomères résiduels inférieures à 0,5 % en poids et une teneur en NCO inférieure à 1 % en poids, dans lequel
à partir
A) de composés contenant des groupes isocyanate,
B) de composés à fonction hydroxy, qui comportent des groupes réagissant avec polymérisation, sous l'effet d'un rayonnement actinique, avec des composés à insaturation éthylénique (groupes durcissables par irradiation) et
C) éventuellement d'autres composés à groupes réactifs avec NCO
D) éventuellement en présence d'un catalyseur
sont formés des uréthannes contenant des groupes NCO, comportant des groupes durcissables par irradiation, qui sont ensuite mis en réaction, sans nouvelle addition de composés contenant des groupes isocyanate, en présence
E) d'un catalyseur d'allophanatisation,
Le rapport des groupes NCO des composés de A) aux groupes OH des composés de B) et éventuellement C) valant de 1,45:1,0 à 1,1:1,0.

2. Procédé pour la préparation d'allophanates durcissables par irradiation selon la revendication 1, **caractérisé en ce que** dans le composant A) on utilise l'hexaméthylène-diisocyanate (HDI), l'isophorone-diisocyanate (IPDI) et/ou le 4,4'-diisocyanatodicyclo-hexylméthane.

3. Procédé pour la préparation d'allophanates durcissables par irradiation selon la revendication 1 ou 2, **caractérisé en ce que** dans le composant B) on utilise le (méth)acrylate d'hydroxyéthyle, le (méth)acrylate d'hydroxypropyle et/ou le (méth)acrylate d'hydroxybutyle.

4. Procédé pour la préparation d'allophanates durcissables par irradiation selon l'une quelconque des revendications 1 à 3, **caractérisé en ce que** le rapport des groupes NCO des composés de A) aux groupes OH des composés de B) et éventuellement C) vaut de 1,35:1,0 à 1,3:1,0.

5. Procédé pour la préparation d'allophanates durcissables par irradiation selon l'une quelconque des revendications 1 à 4, **caractérisé en ce qu'**on ajoute le catalyseur E) à une vitesse de 200 - 600 ppm/h.

6. Procédé pour la préparation d'allophanates durcissables par irradiation selon l'une quelconque des revendications 1 à 5, **caractérisé en ce qu'**on effectue l'allophanatisation jusqu'à ce que le produit final ait une teneur en NCO de moins de 0,1 % en poids.

7. Allophanates durcissables par irradiation, pouvant être obtenus conformément à un procédé selon l'une quelconque des revendications 1 à 6.

8. Utilisation des allophanates durcissables par irradiation selon la revendication 7, dans la production de revêtements et de peintures ainsi que d'adhésifs, d'encres d'impression, de résines de coulée, de matières dentaires, de colles pour encollage, de photoresists, de systèmes de stéréolithographie, de résines pour matériaux composites et de matières d'étanchéité.

9. Composition de revêtement, contenant
a) un ou plusieurs des allophanates durcissables par irradiation selon la revendication 7.
b) éventuellement un ou plusieurs polyisocyanates à groupes isocyanate libres ou bloqués, qui sont exempts de groupes réagissant avec polymérisation, sous l'effet d'un rayonnement actinique, avec des composés à insaturation éthylénique,
c) éventuellement d'autres composés différents de a), qui comportent des groupes réagissant avec polymérisation, sous l'effet d'un rayonnement actinique, avec des composés à insaturation éthylénique, et éventuellement des groupes NCO libres ou bloqués,
d) éventuellement un ou plusieurs composés contenant de l'hydrogène actif, réagissant avec des isocyanates,
e) des amorceurs,
f) éventuellement des solvants et
g) éventuellement des adjuvants et additifs.

10. Supports revêtus avec des revêtements pouvant être obtenus avec utilisation d'allophanates durcissables par irradiation selon la revendication 7.
